# EUROPEAN PATENT APPLICATION

(11) **EP 4 699 519 A1**
(43) Date of publication of application: **25.02.2026**
(21) Application number: 24814591.4
(22) Date of filing: 31.05.2024
(51) Int. Cl.: A61B 5/00, G01N 33/48, G01N 27/22

(54) **MEASUREMENT APPARATUS FOR SECRETED SUBSTANCE OF VITAL BODY**

(30) Priority: 02.06.2023 CN 202310646526; 02.06.2023 CN 202321387855 U
(71) Applicant: Beijing Tashan Technology Co., Ltd., Beijing 102308 (CN)
(72) Inventor: SUN, Tengchen, Beijing 102308 (CN); ZENG, Fanyou, Beijing 102308 (CN); WANG, Zhen, Beijing 102308 (CN)
(74) Representative: Yang, Shu
(86) International application number: PCT/CN2024/096662
(87) International publication number: WO 2024/245397

(57) **Abstract**

The present invention relates to a device for measuring secretion substances of a living organism, comprising an absorption membrane, an electrode detection component, a capacitance-to-digital conversion circuit (CDC) , and a processing module; the absorption membrane is used to absorb secretion substances of a living organism ; the capacitance-to-digital conversion circuit is coupled with the electrode detection component, and is used to detect secretion substances of a living organism through the electrode detection component.

Obtaining the mutual capacitance when the absorption membrane has secreted substances; the processing module is coupled to the capacitance-to-digital conversion circuit, and the processing module is used to obtain the mutual capacitance when the absorption membrane does not have secreted substances, and output the content signal of the substance secreted by the living organism based on the two obtained mutual capacitances.

## Description

### Technical Field

The present invention relates to the measurement of secretory substances of a living organism, in particular to the measurement of the oil content secreted by human skin.

### Background Art

Traditional methods for detecting oil in the skin often use thin film measurement combined with optical measurement. The thin film absorbs oil and then performs optical comparisons with standards. For example, U.S. Patent 4,532,937 discloses a microporous membrane adhered to the skin for sebum absorption. U.S. Patent 5,119,828 discloses the use of a microporous hydrophobic polymer membrane. When the pores are filled with a gaseous material, the membrane is opaque, but when the pores are filled with sebum, the membrane becomes translucent, utilizing this property for optical measurement. Alternatively, German Patent DE29700324U1 discloses the use of a test membrane for skin analysis and evaluation.

The most commonly used device for skin oil detection in the beauty or medical field is the CK device, which also uses oil-absorbing paper + photoelectric type. Each operation requires the detection end to be inserted into another component for calibration.

( The oil measurement device is divided into two parts ), and then the end is placed in contact with the measured area for 30 seconds before being inserted back into the other part for measurement. The operation is complicated and inefficient, and the oil content of the skin is indirectly reflected through light transmittance .

The actual unit of measurement for epidermal oil content is micrograms per square centimeter ( ug/cm² ). The CK device can only distinguish oil content values between 50 and 350ug/cm² based on the transmittance of oil-absorbing paper .

This roughly classifies different areas of the body into three levels: low oil content, normal oil content, and high oil content. The corresponding table is as follows:

| / | Forehed,Tshapedarea of face,scap | hair | Cheeks,eyelids, sunHoles,temples | Corners of mouth, upper body, Back and neck | Arms,han ds,Legs, elbows |
|---|---|---|---|---|---|
| Less oil | <100 | <40 | <70 | <55 | 0-6 |
| norma I | 100-180 | 40-100 | 70-150 | 55-130 | >6 |
| Oily | >180 | >100 | >150 | >130 | / |

The characteristics of the oil-absorbing paper material itself and the area of grease spread on the oil-absorbing paper affect the light transmittance.

The relationship between oil-absorbing paper's transmittance and oil content is influenced by numerous factors, placing high demands on the oil-absorbing paper (consumables), requiring specialized, specialized oil-absorbing paper. Therefore, whether using traditional technology or CK equipment, the measured values from thin-film + optical techniques cannot accurately correlate with oil content, resulting in a wide ambiguity and, in theory, limited room for improvement in accuracy.

### Summary of the Invention

The present invention aims to improve the shortcomings of the prior art and proposes a device for measuring secretions of living organisms. The device comprises an absorption membrane, an electrode detection component, a capacitor, and a
A digital-to-digital conversion circuit (CDC) and a processing module; the absorption membrane is used to absorb secretions of a living organism ; the capacitance-to-digital conversion circuit is coupled with an electrode detection component, and is used to obtain, via the electrode detection component , the mutual capacitance when the absorption membrane has secretions; the processing module is coupled with the capacitance-to-digital conversion circuit, and the processing module is used to obtain the mutual capacitance when the absorption membrane does not have secretions, and output a content signal of the substance secreted by the living organism based on the two obtained mutual capacitances.

The measuring device of the present invention can measure the content of various substances secreted by living organisms through mutual capacitance . For example, using oil secretion from the skin as an example, the absorbing membrane is oil-absorbing paper. By directly detecting the change in mutual capacitance when the oil-absorbing paper is present or absent, this incremental change directly due to the amount of oil present essentially eliminates the influence of the oil-absorbing paper's material itself and is directly related only to the amount of oil present. The main components of oil secreted by living organisms, especially the human face, are triglycerides, fatty acids, phospholipids, inositol, and lipofectamine. The combined dielectric constant of most oils is between 3.04 and 3.2 , while the dielectric constant of PP, the main component of oil-absorbing paper, is 2.3-2.6 . According to the capacitance formula C = ε S/d , capacitance and dielectric constant are directly proportional. It can be seen that the dielectric constant of oil is generally about 30% greater than that of oil-absorbing paper , resulting in a more significant incremental change in capacitance. Therefore, it is possible to accurately measure the oil content on the oil-absorbing paper. In addition, with the help of capacitance-to-digital converter (CDC) , such as DAI7142 and ADI7147 , the Δ-Σ modulation method is used to charge and discharge the measured capacitance multiple times and compare it with the reference capacitance ( see: US Patent Number : 5,134,401) directly converts the measured capacitance value into a digital value, which can increase the measurement sensitivity of the capacitance to 1ff level.

The measuring device of the present invention can immediately detect the area to be measured after the detection end contacts the area to be measured for a certain period of time.

It can provide feedback on the oil content of the skin at one time, and the equipment is simple, integrated and low-cost.

For the mutual capacitance with and without secretion on the absorbent membrane, there are three methods, divided by the time interval between the two mutual capacitances, from far to near: (1) pre-calibration; (2) time -sharing detection; (3) simultaneous detection . The longer the time interval, the greater the difference in the measurement environment, resulting in a decrease in accuracy. For the pre-calibrated solution, the hardware configuration of the measuring device also includes a measuring surface, which is configured to indirectly contact the living organism through the absorption membrane; the electrode detection component is configured to include at least two first electrodes provided on the measuring surface; the absorption membrane is placed on the measuring surface during measurement and is located in the mutual capacitance detection space formed between the first electrodes; the capacitance-to-digital conversion circuit is coupled to each first electrode to obtain a first mutual capacitance between the first electrodes; and the processing module is coupled to the capacitance-to-digital conversion circuit. Based on the above hardware structure, the mutual capacitance data when there is no secretion substance on the absorption membrane can be detected in advance and stored in the memory of the processing module for use as a reference data. During detection, the first mutual capacitance after the absorption membrane absorbs the secretion substance is obtained through the first electrode. The processing module outputs a content signal of the substance secreted by the living organism based on the reference data and the first mutual capacitance after the absorption membrane absorbs the secretion substance. For example, the reference data reflects the dielectric constant of the absorption membrane alone, and the mutual capacitance after the absorption membrane absorbs the secretion substance reflects the dielectric constant of the absorption membrane and the secretion substance. The difference between the two values can be used to highlight the secretion substance and thus perform capacitance detection on its content. For the pre-calibration solution, since the time interval between obtaining the two mutual capacitances is relatively long, the interference caused by the difference in environmental changes will be relatively large.

The time-sharing detection solution also relies on the aforementioned hardware structure. During detection, human-computer interaction, such as clicking a button, is used to measure the first mutual capacitance of the absorption membrane placed on the measurement surface before absorbing secretions. The absorption membrane is then placed in contact with the surface of the living organism for a period of time, and the button is clicked again to measure the first mutual capacitance of the absorption membrane after absorbing the secretions. The processing module outputs a signal indicating the content of the substance secreted by the living organism based on the first mutual capacitance before and after the absorption of the secretions by the absorption membrane. In this solution, since the time interval between the two mutual capacitances is short (on the order of seconds), interference from environmental changes is low. Furthermore, for example, the difference between the two mutual capacitances is used to subtract the common-mode interference of the environment (mutual capacitance after absorption, Caw 2 + C 2) .

Environmental common-mode interference - mutual capacitance before absorption C aw 1 + C Environmental common mode interference) to obtain more accurate detection results.

For the simultaneous detection scheme, on the basis of the above hardware structure, a non-measurement surface needs to be set up for During measurement, the absorption membrane is in non-contact with the living organism. The electrode detection assembly is further configured to include at least two second electrodes disposed on the non-measurement surface. The absorption membrane is synchronously positioned on the non-measurement surface during measurement and located within a mutual capacitance detection space formed between the second electrodes. A capacitance-to-digital conversion circuit is coupled to each second electrode to obtain a second mutual capacitance between the second electrodes. Under the aforementioned hardware structure, during detection, the second mutual capacitance is used as the mutual capacitance when the absorption membrane is free of secreted material, while the first mutual capacitance after the absorption membrane absorbs the secreted material is used as the mutual capacitance when the absorption membrane is present. The processing module outputs a signal indicating the content of the substance secreted by the living organism based on the second mutual capacitance and the first mutual capacitance after the absorption membrane absorbs the secreted material. In this solution, the acquisition interval between the two capacitances is nearly simultaneous (milliseconds, depending on the chip sampling interval), substantially eliminating interference from environmental fluctuations. Furthermore, a difference calculation can subtract errors caused by common-mode environmental interference, resulting in more accurate detection. Since simultaneous detection is performed in two regions, to avoid inherent interference caused by differences in the shapes and sizes of the electrodes (first and second electrodes) in the two regions (different shapes and sizes of electrodes will result in differences in mutual capacitance electric fields, and even for the same substance, the obtained mutual capacitance will be different), the shapes and sizes of the electrodes in the two regions can be set to be consistent. However, in practice, due to manufacturing process issues, theoretical consistency is basically impossible to achieve. To this end, the content signal can be corrected based on the second mutual capacitance. Specifically, the correction method further includes: obtaining a first difference between the first and second mutual capacitances before the absorption membrane absorbs the secreted substance; obtaining a second difference between the first and second mutual capacitances after the absorption membrane absorbs the secreted substance; and a processing module outputting a content signal of the substance secreted by the living organism based on the difference between the first and second differences. Before the absorption membrane absorbs the secretion substance, there is no secretion substance on the absorption membrane. The first difference after subtraction reflects the inherent interference of the electrode. After the absorption membrane absorbs the secretion substance, one of the absorption membranes in the two areas has the secretion substance and the other does not. The second difference after subtraction reflects the dielectric constant of the secretion substance and the inherent interference of the electrode. By subtracting the first difference from the second difference, the inherent interference of the electrode can be removed, and the dielectric constant of the secretion substance can be obtained for detection. In this scheme, there is no need to ensure that the electrodes in the two areas must be consistent, the precision requirements for electrode manufacturing are not strict, and the manufacturing cost can be reduced.

As an improvement, an insulating layer can be provided on the surface of each electrode to reduce the interference with the capacitor.

interference, while protecting the electrodes from corrosion by secretions.

Since the absorption film has a significant impact on the detection of the dielectric constant depending on whether it is tightly attached to the various surfaces being measured, as another improvement, a tightening device can be provided on the measuring device to tighten the absorption film so that it is tightly attached to the corresponding surface during measurement. Furthermore, the tightening device can be configured to include a fixing component and a rotating shaft knob, respectively provided on both sides of the measuring surface. The fixing component fixes one end of the absorption film, and the other end of the absorption film passes through the axial hole in the rotating shaft knob. After rotating the rotating shaft, the knurling on the side wall of the rotating shaft is tightened to achieve tightening. The fixing component is configured as a single-end spring compression fixing component. Due to the easy operation of the single-end spring compression fixing component, the absorption film can be removed by applying force to the pressing wrench, and the absorption film can be tightened by releasing the pressing wrench. During use, the absorption membrane is put in from the entrance, and one end of the absorption membrane is fixed by the single-end spring compression fixing assembly. The other end of the absorption membrane bypasses the electrode detection elastic assembly and is put into the other end of the absorption membrane from the entrance, and passes through the central axis hole of the shaft knob. The shaft is rotated, and the friction between the knurling on the side wall of the shaft and the absorption membrane is increased to tighten the tail end of the absorption membrane until the electrode detection elastic assembly is pressed down to the limit bottom, and then the card pin is pushed into the inner tooth groove to fix it, ensuring that the electrode detection assembly has a fixed thrust on the absorption membrane through the rebound force of the spring, so that the absorption membrane is tightened.

As another improvement scheme, a capacitance-to-digital conversion circuit can be set up to obtain self-capacitance through the electrode detection component. The self-capacitance can reflect the distance of the absorption membrane from the measured surface and thus reflect the degree of tightness. At this time, the processing module can output a logical signal that the absorption membrane is separated from the living organism based on the self-capacitance (through threshold comparison) , and / or the processing module can correct the obtained mutual capacitance based on the self-capacitance to avoid interference due to different tightness.
the spacing between the electrodes ( for example, between the first electrodes ) used to detect the mutual capacitance in the electrode detection assembly is configured to be between 0.05-0.5 mm . The purpose is to control the height of the mutual capacitance electric field so that when the absorption film is placed on the measurement surface, the electric field lines can just pass through the absorption film but not pass through its top surface, so that the detection only obtains the dielectric of the material in the absorption film, avoids the dielectric introducing air from the passing part , and improves the detection accuracy.

### BRIEF DESCRIPTION OF THE DRAWINGS

FIG1 shows a front view of a device for measuring secretions of living organisms ;
FIG2 shows a cross-sectional view of a device for measuring secretion substances of a living organism ;
FIG3 shows a cross-sectional view of the electrode assembly;
FIG4 shows a cross-sectional view of the rotating shaft tensioning assembly and the spring compression assembly;
Figure 5 shows a schematic diagram of the distribution of detection electrodes;
Figure 6 shows a schematic diagram of the stacked structure of the detection electrodes and the absorption membrane.

The technical solutions in the embodiments of the present invention will be described clearly and completely below with reference to the accompanying drawings in the embodiments of the present invention.

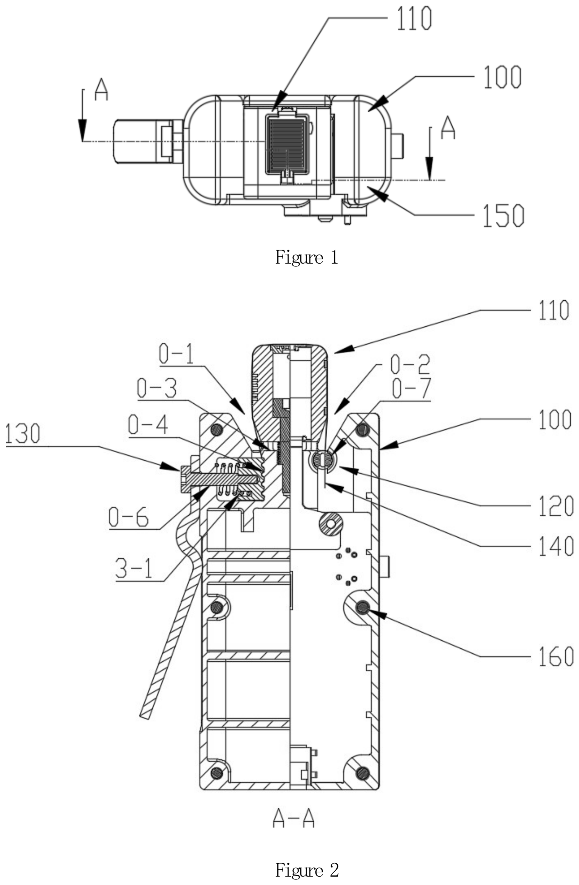

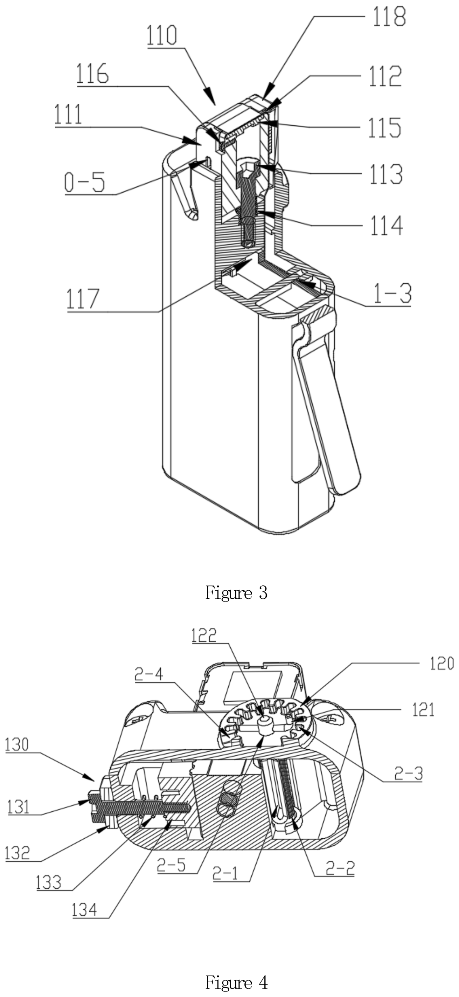

Figures 1 , 2 , 3 , 4 , and 5, the device for measuring secretion substances of living organisms includes a lower cover 100, an absorption membrane 140, an electrode detection assembly 110, a rotating shaft tensioning assembly 120, a spring compression assembly 130, an upper cover 150, and a fastening bolt 160. The electrode detection assembly includes a head 111, an FPCA circuit assembly 112 , a guide bolt 113, a spring 114, a cover bracket 115, a plastic bolt 116, a PFBA circuit assembly 117 , and a circuit protection cover 118; the guide bolt 113 passes through the head 111 and the spring 114 and is screwed together with the lower cover 100, and is limited by the head 111 and the rib groove 0-5 of the lower cover 100 to prevent deflection; one end of the FPCA circuit assembly 112 is attached to the upper cover 100.

On the side of the top head 111 , electrodes 1-1 and 1-2 are bent and attached to the top and side surfaces of the top head 111 respectively to prevent the FPC soft board from warping due to bending stress. Ears extend from the end of the electrode, and plastic bolts 116 pass through the ears at the end of the electrode and are screwed together with the top head 111. The other end of the FPCA circuit assembly is welded to the PCBA short-circuit assembly 117 and positioned and assembled inside the lower cover 100 shell through the hole column 1-3 . The circuit protection cover 118 and the top head 111 are then fastened together through the buckles 1-4. The spring clamping assembly 130 includes a guide bolt 131, a pressure plate 132, a spring 133, and a toothed block 134. The spring 133 is placed in the spring slot 3-1 of the toothed block 134, and then the toothed block 134 with the spring 133 installed is assembled tightly with the lower cover 100 through the teeth 0-4 . At this time, the guide bolt 131

Through the pressing piece 132 and the side hole 0-6 of the lower cover 100 and the toothed block 134, tighten it.

120 includes a card pin 121 and a shaft 122 ; the shaft 122 is assembled at hole position 0-7 of the lower cover 100, and the upper cover 150
The bottom cover 100 is positioned and assembled through the hole column 1-3 , and the top of the shaft 122 passes through the limiting hole 2-5 of the top cover 150.

The card pin 121 passes through the guide groove 2-4 and is inserted into the top side hole of the rotating shaft 122. At this time, the upper and lower covers are fastened by the bolts 160.

Secure and lock.

Test preparation: by entrance 0 - 1 Place the absorbent membrane 14 0 , springloaded assembly 13 0 Fixed absorbent membrane 140
One end; the other end absorbs the membrane 140 Bypass electrode detection component 110 From entrance 0-2 Insert and pass through the shaft
122 middle axis hole 2-1 , rotate the card pin 121 to make the shaft 122 Rotate, through the knurling of the shaft side wall 2-2 With the absorption film 140 Increase friction to tighten it until the electrode detection component 110 Press down to the limit bottom 0-3 , then insert the card pin 121 Push to the inner tooth groove 2-3 Fixed in the middle to ensure that the electrode detection component 110 By Spring 114 The elastic force

The suction film 140 has a fixed thrust, which keeps the suction film 140 in a standard stretched state.

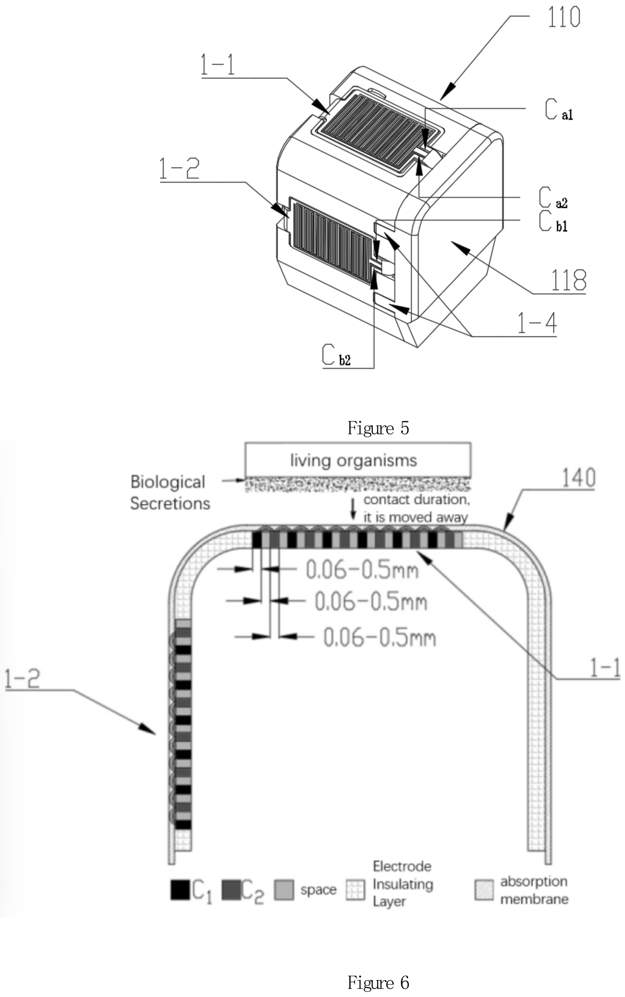

In FIG5, the electrode detection assembly 110 includes a first electrode group 1-1 and a second electrode group 1-2. Each electrode group is composed of two intersecting comb-shaped electrodes, and the two electrode groups have synchronized shapes. The first electrode group includes electrodes Ca1 and Ca2 , configured as a first mutual capacitance Caw, and the second electrode group includes electrodes Cb1 and Cb2 , configured as a second mutual capacitance Cbw . Figures 5 and 6, the first electrode group 1-1 contacts the living organism and absorbs secretions, while the second electrode group 1-2 serves as a reference for comparison with the first electrode group 1-1 and does not contact the living organism. The electrode surface is covered with an insulating film, and the electrode line width and the spacing between the electrodes are kept within the range of 0.05-0.5mm to ensure that the mutual capacitance electric field covers the absorption membrane 140 of various models and thicknesses without exceeding the outer surface of the absorption membrane.

For the mutual capacitance when there is and when there is no secretion substance on the absorption membrane 140, the operation sequence is as follows:
Pre-calibration measurement method: Assume that the absorption membrane 140 is not absorbing the secretion of the living organism; then start the test to obtain the first set of mutual capacitance values CaW1, and set the first set of test mutual capacitance values CaW1 as the baseline data; then the living organism approaches and contacts the first electrode group 1-1 , and the surface absorption membrane 140
140 leaves after the set contact time. At this time, the membrane 140 has absorbed the secretion substances of the living organism, and the second set of mutual capacitance values Caw 2 is obtained again. The difference between Caw 2 and CaW 1 is used as the capacitance value of the secretion substances in the membrane. When the same standard membrane 140 is subsequently replaced , the capacitance value before absorbing the secretion substances is no longer tested, and only the first set of test capacitance data Caw 1 is used as the comparison benchmark value.

Time-sharing detection method: assume that the absorption membrane 140 is currently in a state where it is not absorbing the secretion of the living organism; at this time, the test begins to obtain the first set of mutual capacitance values CaW1 , and then the living organism approaches and contacts the first electrode group 1-1. At this time, the surface absorption membrane 140 and the secretion of the living organism merge and absorb. After the set contact time ends,

After leaving, the absorption membrane 140 has absorbed the secretion substances of the living organisms. The second set of mutual capacitance values C aw 2 is obtained by testing again . The difference between C aw 2 and CaW 1 is used as the capacitance value of the secretion substances in the absorption membrane. Subsequent replacement of the absorption membrane 140 requires the synchronization of the above operation steps.

Simultaneous detection method: Assume that the absorption membrane 140 is currently in a state where it has not absorbed the secretion of the living organism; at this time, the living organism directly approaches and contacts the first electrode group 1 - 1 , at this time the surface absorbs the membrane 140 After the contact time is set, the membrane 140 is separated from the living organisms and the first set of mutual capacitance values CaW 1 and CbW 1 are obtained through testing. The difference between Caw 1 and CbW 1 is used as the capacitance value of the secretion substance in the absorption membrane. Subsequent replacement of the absorption membrane 140 requires synchronization with the above steps.

Finally, it should be noted that the above embodiments are only used to illustrate the technical solutions of the present invention, rather than to limit the scope of protection of the present invention. Although the present invention has been described in detail with reference to the preferred embodiments, those skilled in the art should understand that the technical solutions of the present invention may be modified or replaced by equivalents without departing from the essence and scope of the technical solutions of the present invention.

## Claims

1. A device for measuring secretion substances of a living organism, **characterized in that**:
comprising an absorption membrane (140), an electrode detection component (110), a capacitance-to-digital conversion circuit, and a processing module;
the absorption membrane (140) is configured to absorb secretions of the living organism;
the capacitance-to-digital conversion circuit is coupled to the electrode detection component (110), and is configured to obtain a mutual capacitance when the secretion substance is present on the absorption membrane (140) via the electrode detection component (110);
the processing module is coupled to the capacitance-to-digital conversion circuit, and is configured to obtain the mutual capacitance when the absorption membrane (140) does not have the secretion substance, and output a content signal of the substance secreted by the living organism based on the two obtained mutual capacitances.

2. The device for measuring secretion substances of a living organism according to claim 1, further comprising a measuring surface configured to indirectly contact the living organism via the absorption membrane (140);
the electrode detection component (110) is configured to include at least two first electrodes arranged on the measuring surface;
the absorption membrane (140) is placed on the measuring surface during measurement and is located in a mutual capacitance detection space formed between the first electrodes;
the capacitance-to-digital conversion circuit is coupled to each of the first electrodes, and is configured to obtain a first mutual capacitance (Caw) between the first electrodes;
the processing module is coupled to the capacitance-to-digital conversion circuit.

3. The device for measuring secretion substances of a living organism according to claim 2, **characterized in that**:
the first mutual capacitance (Caw) before the absorption membrane (140) absorbs the secretion substance is used as the mutual capacitance when the absorption membrane (140) does not have the secretion substance;
the first mutual capacitance (Caw) after the absorption membrane (140)** absorbs the secretion substance is used as the mutual capacitance when the **absorption membrane (140)** has the secretion substance;
the processing module outputs a content signal of the substance secreted by the living organism based on the first mutual capacitance (Caw) before and after the absorption membrane (140) absorbs the secretion substance.

4. The device for measuring secretion substances of a living organism according to claim 2, **characterized in that**:
the mutual capacitance when there is no secretion substance on the absorption membrane (140) is detected in advance and stored in a memory of the processing module as benchmark data;
the first mutual capacitance (Caw) after the absorption membrane (140) absorbs the secretion substance is used as the mutual capacitance when the absorption membrane (140) has the secretion substance;
the processing module outputs a content signal of the substance secreted by the living organism based on the benchmark data and the first mutual capacitance (Caw) after the absorption membrane (140) absorbs the secretion substance.

5. The device for measuring secretion substances of a living organism according to claim 2, **characterized in that**:
the device further comprises a non-measuring surface, for which the absorption membrane (140) is not in contact with the living organism during measurement;
the electrode detection component (110) is configured to include at least two second electrodes disposed on the non-measuring surface;
the absorption membrane (140) is synchronously disposed on the non-measuring surface and is located in a mutual capacitance detection space formed between the second electrodes during measurement;
the capacitance-to-digital conversion circuit is coupled to each of the second electrodes and is configured to obtain a second mutual capacitance (Cbw) between the second electrodes.

6. The device for measuring secretion substances of a living organism according to claim 5, wherein:
the second mutual capacitance (Cbw) is used as the mutual capacitance when the absorption membrane (140) is free of the secretion substance;
the first mutual capacitance (Caw) after the absorption membrane (140) absorbs the secretion substance is used as the mutual capacitance when the absorption membrane (140) has the secretion substance;
the processing module outputs a content signal of the substance secreted by the living organism based on the second mutual capacitance (Cbw) and the first mutual capacitance (Caw) after the absorption membrane (140) absorbs the secretion substance.

7. The device for measuring secretion substances of a living organism according to claim 6, wherein the processing module corrects the content signal based on the second mutual capacitance (Cbw).

8. The device for measuring secretion substances of a living organism according to claim 7, wherein the correction method further comprises:
before the absorption membrane (140) absorbs the secretion substance, obtaining a first difference between the first mutual capacitance (Caw) and the second mutual capacitance (Cbw);
after the absorption membrane (140) absorbs the secretion substance, obtaining a second difference between the first mutual capacitance (Caw) and the second mutual capacitance (Cbw);
the processing module outputs a content signal of a substance secreted by the living organism based on a difference between the first difference and the second difference.

9. The device for measuring secretion substances of a living organism according to claim 2 or 5, wherein an insulating layer is provided on a surface of each electrode.

10. The device for measuring secretion substances of a living organism according to claim 2 or 5, further comprising a tightening device configured to tighten the absorption membrane (140) to make it adhere closely to a corresponding surface during measurement.

11. The device for measuring secretion substances of a living organism according to claim 10, wherein the tightening device comprises a fixing assembly (130) and a rotating shaft knob (122) respectively arranged on both sides of the measuring surface, the fixing assembly (130)
The component fixes one end of the absorption film, and the other end of the absorption film passes through the shaft hole (2-1) in the rotating shaft knob (122, and after the rotating shaft (122) is rotated, the knurling (2-2) on the side wall of the rotating shaft is tightened to achieve the tightening.

12. The device for measuring secretion substances of a living organism according to claim 11, wherein the fixing component (130) is configured as a single-end springloaded fixing component.

13. The device for measuring secretion substances of a living organism according to claim 1, wherein the capacitance-to-digital conversion circuit is further configured to obtain a self-capacitance via the electrode detection component (110);
the processing module is configured to output a logic signal indicating that the absorption membrane (140) is separated from the living organism based on the self-capacitance, and/or to correct the acquired mutual capacitance based on the self-capacitance.

14. The device for measuring secretion substances of a living organism according to claim 1, wherein the secretion substance is configured as oil, and the absorption membrane (140) is configured as oil-absorbing paper.
